# EUROPEAN PATENT APPLICATION

(11) **EP 1 293 202 A1**
(43) Date of publication of application: **19.03.2003**
(21) Application number: 01904367.8
(22) Date of filing: 09.02.2001
(51) Int. Cl.: A61K 31/166, A61P 19/02

(54) **REMEDIES FOR DEGENERATIVE ARTHRITIS**

(30) Priority: 10.02.2000 JP 2000033422
(71) Applicant: ONO PHARMACEUTICAL CO., LTD., Osaka-shi, Osaka 541-8526 (JP)
(72) Inventor: MAEDA, Yoshizou Minase Res.Inst. ONO Pharma.Co.Ltd, Mishima-gun, Osaka 618-8585 (JP); YONETOMI, Yasuo Minase Res.Inst. ONO Pharma.Co.Ltd, Mishima-gun, Osaka 618-8585 (JP); OGAWA, Koji Minase Res. Inst. ONO Pharma. Co. Ltd, Mishima-gun, Osaka 618-8585 (JP)
(74) Representative: Henkel, Feiler, Hänzel
(86) International application number: JP0100914
(87) International publication number: WO01058440

(57) **Abstract**

A treatment and/or prevention agent for osteoarthritis comprising N-hydroxy-5-ethoxymethyloxy-2(S)-methyl-4(S)-(4-phenoxybenzoyl)amino pentanamide having an inhibitory activity of matrix metalloproteinases, or a non-toxic salt thereof as active ingredient.

## Description

### Summary

This invention relates to a treatment and/or prevention agent for osteoarthritis. More particularly, this invention relates to a treatment and/or prevention agent for osteoarthritis comprising N-hydroxy-5-ethoxymethyloxy-2(S)-methyl-4(S)-(4-phenoxybenzoyl)aminopentanamide having an inhibitory activity of matrix metalloproteinases, or a non-toxic salt thereof as active ingredient.

### Background

The matrix metalloproteinases (MMPs) are neutral metalloproteinases and zinc (Zn²⁺) is essential in the active site for their activation. At least 20 classes of MMPs, which differ in primary structure, are identified. Concretely, there are Interstitial Collagenase (MMP-1), Neutrophil Collagenase (MMP-8), Collagenase-3 (MMP-13), Gelatinase A (MMP-2), Gelatinase B (MMP-9), Stromelysin-1 (MMP-3), Stromelysin-2 (MMP-10), Matrilysin (MMP-7), metalloelastase (MMP-12) etc.

They degrade collagen, laminin, proteoglycans, fibronectin, elastin, gelatin etc. under physiological conditions and therefore, are effective on growth and tissue remodeling of articulation tissue, bone tissue and connective tissue. However, it is consider that a disorganization of various tissues under morbid condition was caused by an increase of an expression or an activity of MMPs which was depend to a bankruptcy of MMP modulation. For example, it was reported that MMP-1, 2, 3, 8, 9, 13 was high-expression in a part of damaged cartilage of patients with osteoarthritis, and these MMPs were deeply concerned with a degradation of cartilage [Igaku no ayumi, 182, 549-553 (1997); J. Clin. Invest., 84, 678-685 (1989)].

On the other hand, osteoarthritis (OA) is at the top of arthralgia for middle-aged and elderly patients. A decrease of resiliency, abrasion, fissure was caused due to a degenerative change of articular cartilage, and so a chondral ossification was increased and new bone forming around a joint was encouraged, at last, an articular cartilage was disappeared and so a bone was outcropped at a joint, and arthralgia and pooling fluid of a joint were caused. A favorite site was knee, crotch, spinal cord, cubitus, shoulder or finger. It was often the case that a cause was not known.
Non-steroidal analgesic and anti-inflammatory drug were used as a first-line drug for the above disease. Besides, an injection of hyaluronic acid preparation which having an increase ability of lubrication for a joint and anti-inflammatory activity, to a joint was carried out. However, there are nothing but a stopgap treatment, and it was not established a fundamental treatment.

For example, in the specification of GB2268934 and EP606046, OA was described as a disease, which is susceptible to treatment of a compound having MMP inhibitory activity. CGS-27023A: 2(R)-[N-(4-methoxyphenylsulfonyl)-N-(3-pyridylmethyl)amino]-3-methylbutanohydroxamic acid hydrochloride; which was MMP inhibitor, was reported to be effected on a model of OA in rabbits [Inflamm Res 44, Supplemet 2, S117-118 (1995)]. However, there are no MMP inhibitors as a treatment and/or prevention agent for OA, and a compound having MMP inhibitory activity under development for OA was precious few.

### Disclosure of the Invention

As previously noted, it was eagerly looking forward to supply a treatment and/or a prevention agent for OA, and it is hoped that MMP inhibitors will be the agents. However, not all known MMP inhibitors are useful for OA, and the fruition has not been achieved.
Energetic investigations have been carried out in order to accomplish the above purpose. The present inventors have found first that N-hydroxy-5-ethoxymethyloxy-2(S)-methyl-4(S)-(4-phenoxybenzoyl)aminopentanamide or a non-toxic salt thereof is useful for OA, and accomplished the present invention.
This invention relates to a treatment and/or prevention agent for osteoarthritis comprising N-hydroxy-5-ethoxymethyloxy-2(S)-methyl-4(S)-(4-phenoxybenzoyl)aminopentanamide of formula (I) 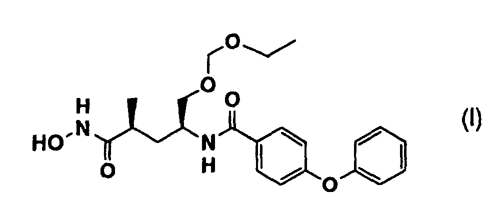 or a non-toxic salt thereof as active ingredient.

N-hydroxy-5-ethoxymethyloxy-2(S)-methyl-4(S)-(4-phenoxybenzoyl) aminopentanamide of formula (I) was described in Example 71 of the specification of WO 99/19296, as an MMP inhibitor. An inhibitory activity of MMP, for example, each value of IC50 (nM) for MMP-2 and MMP-3 was 0.5 and 26.
In the above specification, it was described that aminobutyric acid derivatives containing the compound of formula (I) have MMP inhibitory activity, so they are useful for prevention and / or treatment of diseases, for example, rheumatoid diseases, arthrosteitis, unusual bone resorption, osteoporosis, periodontitis, interstitial nephritis, arteriosclerosis, pulmonary emphysema, cirrhosis, cornea injury, cornea ulcer, metastasis, invasion or growth of tumor cells, autoimmune disease (Crohn's disease, Sjogren's syndrome), disease caused by vascular emigration or infiltration of leukocytes, arterialization, multiple sclerosis, arota aneurysm, endometriosis. However, there is no description that the compound of formula (I) is useful for OA.
Although some kind of relations between MMP and OA were known, the present inventors applied some MMP inhibitors to a partial meniscectomy model of osteoarthritis rabbits, then only the compound of formula (I) shows an efficiency.

### Brief Description of Drawing

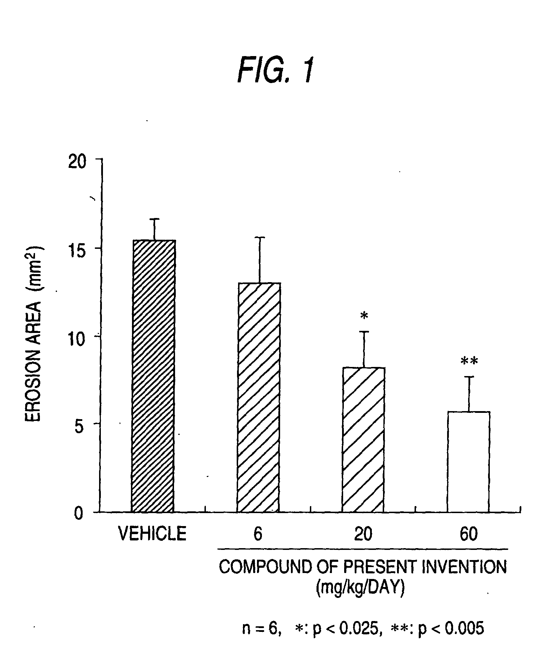

Figure 1 shows a decreasing effect of an erosion area of femur by an administration of the compound of formula (I) at 10, 30, 100 or 300 mg/kg twice a day.

### Detailed description of the Invention

The compounds of the present invention may be converted into the corresponding salts. Non-toxic salts and water-soluble salts are preferred. Suitable salts, for example, include:
salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine).

The compounds of formula (I) and salts thereof may be converted into the corresponding hydrates by conventional manner.

### Best Mode for Carrying out the Invention

The effect of the compound on osteoarthritis invented in this study was confirmed by the experiments of a partial meniscectomy model in rabbits.

### [Method]

Female rabbits (Kbs: NZW(Healthy)) were bred for one week before the experiments. The animals were subjected to OA model according to following method.
Each animal was anesthetized with a subcutaneous injection of xylazine (0.05 ml/kg) and an intravenous injection of sodium pentobarbital (20 mg/kg). Right hind knee was shaved, and the width of the knee was measured with the micrometer caliper. It was disinfected with iodine solution. If required local anesthesia was performed with lidocaine hydrochloride.
The skin and articular capsule around right knee were incised at right angle to patellar ligament, the fibular collateral and sesamoid ligament were transected with blades. Adrenaline solution was dropped for the arrest of hemorrhage. The lateral meniscus was partially removed. After washing the operated area with saline solution, the synovium and articular capsule were stitched together. Then muscular coat and skin were put in a suture.
After operation penicillin G potassium (5000 U/animal) and streptomycin sulfate (100 mg/animal) were administered intramuscularly. Test compound was administered orally twice a day and the animals were sacrificed 7 days after meniscectomy.
After anesthesia with sodium pentobarbital (40 mg/kg), the hind knee was shaved and the width of them was measured with a micrometer caliper. Animals were sacrificed by bleeding, and the right hind knee was obtained. The knee capsule was incised by using scissors and blade, the femur and tibia were obtained. These samples were stored in 10% buffered formalin at room temperature. After masking, the area of erosion was measured.
The values of erosive area among vehicle and compound groups were subjected to statistical analysis using analysis of Williams multiple comparison test. The result was shown in Figure 1.

### Industrial Applicability

### [Effect]

This model can induce a damaged cartilage, which is very like human OA, so that it is acknowledged as OA model. The compound of formula (I) showed high-effectivity on this model, accordingly, it is estimated that the compound of formula (I) is useful for OA.

### Toxicity

The toxicity of the compounds of the present invention is very low and therefore, the compounds may be considered safe for pharmaceutical use. For example, a minimum of lethal dose by single oral administration was 2000 mg/kg.

### Application for Pharmaceuticals

The compound using the present invention, so that N-hydroxy-5-ethoxymethyloxy-2(S)-methyl-4(S)-(4-phenoxybenzoyl)aminopentanamide having an inhibitory activity of matrix metalloproteinase, for example, gelatinase, stromelysin or collagenase, or a non-toxic salt thereof is useful for a treatment and/or prevention of osteoarthritis in animals including human beings, especially human beings.

For the purpose above described, the compounds of present invention, a non-toxic salt thereof or a hydrate thereof may be normally by administered systemically or locally usually by oral or parenteral administration.
The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compound of the present invention may be administered in the form of, for example, solid forms for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.
In such solid forms, one or more of the active compound(s) may be admixed with vehicles (such as lactose, mannitol, glucose, microcrystalline cellulose, starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof.
Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms, for example, freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se. Sprays may comprise additional substances other than diluents, such as stabilizing agents (such as sodium sulfate), isotonic buffers (such as sodium chloride, sodium citrate or citric acid). For preparation of such sprays, for example, the method described in the United States Patent No. 2,868,691 or 3,095,355 may be used.

### Formulation example

### Formulation example 1

The following components were admixed in conventional method and punched out to obtain 100 tablets each containing 50 mg of active ingredient.
· N-hydroxy-5-ethoxymethyloxy-2(S)-methyl-4(S)-(4-phenoxybenzoyl) aminopentanamide 5.0 g
· Carboxymethylcellulose calcium (disintegrating agent) 0.2 g
· Magnesium stearate (lubricating agent) 0.1 g
· Microcrystaline cellulose 4.7 g

### Formulation example 2

The following components were admixed in conventional method. The solution was sterilized in conventional manner, placed 5 ml portions into ampoules and freeze-dried to obtain 100 ampoules each containing 20 mg of the active ingredient.
· N-hydroxy-5-ethoxymethyloxy-2(S)-methyl-4(S)-(4-phenoxybenzoyl) aminopentanamide 2.0 g
· Mannitol 20 g
· Distilled water 500 ml

## Claims

1. A treatment and/or prevention agent for osteoarthritis comprising N-hydroxy-5-ethoxymethyloxy-2(S)-methyl-4(S)-(4-phenoxybenzoyl)amino pentanamide of formula (I) 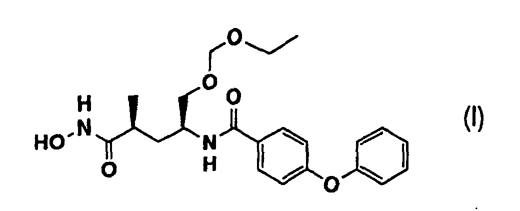 or a non-toxic salt thereof as active ingredient.
